Europäisches Patentamt

European Patent Office

Office européen des brevets

(19)

(11) Numéro de publication : **0 420 709 B1**

(12) **FASCICULE DE BREVET EUROPEEN**

(45) Date de publication du fascicule du brevet :
06.07.94 Bulletin 94/27

(51) Int. Cl.⁵ : **C07C 17/38, C07C 19/08**

(21) Numéro de dépôt : **90402385.0**

(22) Date de dépôt : **29.08.90**

(54) **Purification du dichloro-1,1 fluoro-1 éthane.**

(30) Priorité : **12.09.89 FR 8911882**

(43) Date de publication de la demande :
**03.04.91 Bulletin 91/14**

(45) Mention de la délivrance du brevet :
**06.07.94 Bulletin 94/27**

(84) Etats contractants désignés :
**AT BE CH DE DK ES FR GB GR IT LI LU NL SE**

(56) Documents cités :
**EP-A- 0 399 705**
**EP-A- 0 401 493**
**GB-A- 627 773**
**US-A- 2 560 838**
**US-A- 2 637 747**
**US-A- 4 028 426**

(73) Titulaire : **ELF ATOCHEM S.A.**
**4 & 8, Cours Michelet**
**La Défense 10**
**F-92800 Puteaux (FR)**

(72) Inventeur : **Correia, Yves**
**Les Lauzières**
**F-04160 Chateau-Arnoux (FR)**
Inventeur : **Bergougnan, Michel**
**2 Rue du Docteur Roux**
**F-69310 Pierre-Benite (FR)**
Inventeur : **Lesparre, Jean**
**Quartier Savin**
**F-04290 Volonne (FR)**
Inventeur : **Perdrieux, Sylvain**
**707 Rue de la Maçonnière,**
**Charly**
**F-69390 Vernaison (FR)**

(74) Mandataire : **Leboulenger, Jean et al**
**ATOCHEM**
**Département Propriété Industrielle**
**Cédex 42- La Défense 10**
**F-92091 Paris la Défense (FR)**

## Description

L'invention concerne le domaine des hydrocarbures chlorofluorés et a plus particulièrement pour objet un procédé de purification du dichloro-1,1 fluoro-1 éthane (connu également sous la désignation 141 b).

Ce composé, préparé à partir du dichloro-l,l éthylène (A.E. Feiring, J. Fluorine Chem. 1979, 14(1), 7-18) ou à partir du trichloro-1,1,1 éthane (brevet DE 2 137 806), contient comme impuretés des composés insaturés chlorés ou chlorofluorés qui sont indésirables dans l'utilisation du dichloro-1,1 fluoro-1 éthane. Parmi les impuretés insaturées, les plus gênantes sont le dichloro-1,1 éthylène et le dichloracétylène dont les points d'ébullition (31,7°C et 33°C respectivement) sont très voisins de celui (32°C) du dichloro-1,1 fluoro-1 éthane.

Dans le brevet GB 627 773 relatif à la fabrication de composés organiques fluorés par réaction d'acide fluorhydrique avec des chloro-hydrocarbures insaturés tels que le chlorure de vinyle, le dichloro-1,1 éthylène et le chloro-2 propylène, il est indiqué (exemple 3) que la séparation du produit est facilitée par chloration du dichloro-éthylène non transformé dont le taux de conversion n'est que de 32,7%. Les conditions opératoires de cette chloration ne sont pas indiquées.

La demande de brevet EP-A-399 705 publiée le 28.11.90 décrit un procédé de fabrication du dichloro-1,1 fluoro-1 éthane par addition d'HF au dichloro-1,1 éthylène en présence d'un fluorure métallique. Pour réduire la teneur en dichloro-éthylène, le produit issu d'une première étape d'hydrofluoration en phase gazeuse est soumis à une deuxième étape d'hydrofluoration en phase liquide.

Enfin, la demande de brevet EP-A-401 493 publiée le 12.12.90 décrit un procédé de purification du dichloro-1,1 fluoro-1 éthane par photochloration des impuretés insaturées.

Il a maintenant été trouvé un procédé permettant d'éliminer ces impuretés, principalement le dichloro-1,1 éthylène et le dichloracétylène, et d'obtenir ainsi un dichloro-1,1 fluoro-1 éthane d'excellente pureté.

Le procédé selon l'invention consiste à soumettre le dichloro-1,1 fluoro-1 éthane brut à l'action du chlore et/ou de l'acide chlorhydrique, ce traitement étant réalisé en présence d'un acide de Lewis et éventuellement d'acide fluorhydrique, puis à distiller le produit ainsi traité.

Un dichloro-1,1 fluoro-1 éthane brut à traiter conformément au procédé selon l'invention comprend généralement en poids de 50 à 99,8 % de dichloro-1,1 fluoro-1 éthane, de 0,1 à 0,3 % de dichloro-1,1 éthylène, de 0 à 25 % de trichloro-1,1,1 éthane, et de 0,1 à 25 % de chloro-1 difluoro-1,1 éthane, ainsi qu'à l'état de traces (environ 10-20 ppm) des composés insaturés tel que le dichloroacétylène ; il contient en outre des acides chlorhydrique et fluorhydrique dissous. Bien entendu, on ne sortirait pas du cadre de la présente invention en appliquant ce traitement à un produit brut contenant moins de 0,1 % de dichloro-1,1 éthylène ou en contenant jusqu'à environ 10 %.

Dans la suite de la description, les conditions opératoires sont définies par rapport au dichloro-1,1 éthylène, cette impureté étant la plus importante.

La quantité de chlore et/ou d'acide chlorhydrique à mettre en oeuvre dépend en premier lieu de la teneur en dichloro-1,1 éthylène dans le dichloro-1,1 fluoro-1 éthane brut à traiter. Elle dépend aussi de l'acide de Lewis employé comme catalyseur ; il est en effet connu qu'un catalyseur d'antimoine, par exemple, n'est actif qu'à l'état de $Sb^{+5}$ et a tendance à être réduit en $Sb^{+3}$ par les oléfines, de sorte qu'une quantité minimale de chlore est nécessaire pour maintenir un tel catalyseur dans sa forme active pour réaliser les réactions de chloration et/ou d'hydrochloration ou hydrofluoration. Le rapport molaire :

$$\frac{\text{chlore et/ou HCl}}{CH_2 = CCl_2}$$

est de préférence au moins égal à 1,2 ; il peut aller jusqu'à 10 et est avantageusement compris entre 3 et 7. Le chlore et/ou l'acide chlorhydrique peut être introduit à l'état gazeux ou liquide, directement dans le produit brut à traiter ou préalablement dissous dans une fraction du produit brut à traiter.

L'opération peut être effectuée à une température allant de -20 à +70°C. Cependant, elle est de préférence réalisée en dessous de 35°C, avantageusement entre -5 et 30°C, de manière à éviter la décomposition du trichloro-1,1,1 éthane éventuellement présent dans le produit brut à traiter et/ou formé au cours du traitement.

On peut opérer à la pression atmosphérique ou sous pression jusqu'à 25 bars, mais industriellement on travaille avantageusement sous une pression comprise entre 5 et 15 bars.

Le traitement selon l'invention est effectué en présence d'un acide de Lewis tel que, par exemple, $FeCl_3$, $SbCl_5$, $TaF_5$, $TiCl_4$, $MoCl_5$, $SnCl_4$, $NbF_5$, etc... La proportion de catalyseur peut varier de 0,001 à 1 % en poids par rapport au produit brut à traiter.

Le procédé selon l'invention peut être mis en oeuvre en discontinu, mais industriellement on préfère opérer en continu. La durée du traitement peut varier dans de larges limites. Elle dépend non seulement de la teneur initiale en dichloro-1,1 éthylène dans le dichloro-1,1 fluoro-1 éthane brut à traiter et des conditions opératoires mises en oeuvre, mais aussi de l'efficacité recherchée (élimination plus ou moins complète du dichloro-1,1 éthylène) qu'on peut contrôler facilement par prélèvement d'échantillons et leur analyse chromatographique.

A titre purement indicatif, l'élimination quasi-complète du dichloro-1,1 éthylène peut être obtenue dans une plage de temps allant de quelques minutes (avec $SbCl_5$) à quelques heures (avec $FeCl_3$).

Le traitement terminé, il suffit, après l'éventuelle séparation du catalyseur, de distiller le produit pour obtenir un dichloro-1,1 fluoro-1 éthane d'excellente pureté. Cette distillation, destinée à séparer le dichloro-1,1 fluoro-1 éthane des composés plus légers ou plus lourds présents initialement et/ou formés durant le traitement, est de préférence réalisée à pression atmosphérique, mais peut l'être également sous pression super- ou subatmosphérique.

Le catalyseur peut être, préalablement à la distillation, séparé par lavage alcalin ou basique ou complexé au moyen d'une base de Lewis telle que, par exemple, un trialkyl- ou triarylphosphate, un alcool, l'eau.

Les exemples suivants illustrent l'invention sans la limiter :

## EXEMPLE 1

Dans un ballon en verre noirci, on place 95 g d'un mélange comprenant 0,81 mole de dichloro-1,1 fluoro-1 éthane et $3,1.10^{-3}$ mole de dichloro-1,1 éthylène. Ce mélange étant à 0°C et sous agitation, on y ajoute brutalement 1,2 g de chlore et 0,25 g de pentachlorure d'antimoine. Dix minutes après cette addition, on prélève un échantillon auquel on ajoute un peu d'arsénite de sodium pour bloquer toute évolution de la réaction jusqu'au dosage effectué par chromatographie gazeuse.

On obtient les résultats suivants :

|  | COMPOSITION (% en poids) initiale | après traitement |
|---|---|---|
| dichloro-1,1 fluoro-1 éthane | 99,68 | 94,53 |
| dichloro-1,1 éthylène | 0,32 | < 0,01 |
| chloro-1 difluoro-1,1 éthane |  | 2,14 |
| trichloro-1,1,1 éthane |  | 2,41 |
| trichloroéthylène |  | 0,14 |
| tétrachloro-1,1,1,2 éthane |  | 0,77 |

Outre la disparition du dichloro-1,1 éthylène, on note une dismutation partielle du dichloro-1,1 fluoro-1 éthane en chloro-1 difluoro-1,1 éthane et en trichloro-1,1,1 éthane, ainsi qu'une légère chloration de ce dernier composé en tétrachloroéthane.

Le dichloro-1,1 fluoro-1 éthane peut alors être facilement séparé des autres constituants par distillation fractionnée du mélange.

## EXEMPLE 2

En opérant dans le même appareillage qu'à l'exemple 1 et à la même température (0°C), on met en oeuvre :
- 82 g d'un mélange comprenant 0,7 mole de dichloro-1,1 fluoro-1 éthane et $2,1.10^{-3}$ mole de dichloro-1,1 éthylène,
- 1 g de chlore,
- 0,5 g de $FeCl_3$

Les analyses chromatographiques effectuées en fonction du temps (t), après blocage de la réaction, ont donné les résultats suivants :

| | COMPOSITION (% poids) au temps t(minute) | | | |
|---|---|---|---|---|
| | t = 0 | t = 30 | t = 60 | t=180 |
| dichloro-1,1 fluoro-1 éthane | 99,75 | 99,66 | 99,64 | 99,64 |
| dichloro-1,1 éthylène | 0,25 | 0,12 | 0,05 | 0,01 |
| chloro-1 difluoro-1,1 éthane | traces | traces | traces | traces |
| trichloroéthylène | | 0 | 0,02 | 0,05 |
| tétrachloro-1,1,1,2 éthane | | 0,22 | 0,29 | 0,30 |

## EXEMPLE 3

On procède dans le même appareillage qu'à l'exemple 1, mais on opère à 29°C environ avec les quantités suivantes :
- 98,07 g d'un mélange comprenant 97,9 g de dichloro-1,1 fluoro-1 éthane et 0,17 g de dichloro-1,1 éthylène,
- 0,8 g de chlore,
- 0,42 g de $FeCl_3$

L'analyse chromatographique effectuée après blocage de la réaction a donné les résultats suivants :

| | COMPOSITION (% en poids) | |
|---|---|---|
| | initiale (t=0) | après traitement (t=30) |
| dichloro-1,1 fluoro-1 éthane | 99,83 | 99,70 |
| dichloro-1,1 éthylène | 0,17 | néant (très inférieur à 0,01) |
| chloro-1 difluoro-1,1 éthane | traces | traces |
| trichloroéthylène | | 0,05 |
| tétrachloro-1,1,1,2 éthane | | 0,25 |

## EXEMPLE 4

Dans un réacteur en verre on place sous agitation à 15-16°C, 500 g de dichloro-1,1 fluoro-1 éthane contenant 46,5 mg de dichloroacétylène, puis on ajoute 800 mg de $SbCl_5$ et 0,3 g de chlore. L'analyse du mélange après 15 minutes montre que le dichloracétylène a entièrement disparu et s'est tranformé principalement en dichloro-1,2 éthylène.

## EXEMPLE 5

Dans un réacteur en verre noirci, maintenu sous agitation et à 15-16°C, on place 2167 g de dichloro-1,1 fluoro-1 éthane industriel, puis on ajoute 600 mg de $FeCl_3$. Au moyen d'un tube plongeur, on introduit ensuite du chlore à un débit tel que la teneur en chlore dissous soit maintenue à environ 5 g/l.

EP 0 420 709 B1

Le tableau suivant donne la composition initiale et celle mesurée après 6 heures de réaction :

| | COMPOSITION (% en poids) | |
|---|---|---|
| | *initiale* | *après 6 heures* |
| dichloro-1,1 fluoro-1 éthane | 89,80 | 86,15 |
| chloro-1 difluoro-1,1 éthane | 1,38 | 1,46 |
| dichloro-1,1 éthylène | 6,00 | ≤ 0,01 |
| trichloro-1,1,1 éthane | 2,32 | 2,40 |
| trichloroéthylène | 0,50 | 0,78 |
| tétrachloro-1,1,1,2 éthane | – | 8,00 |
| pentachloroéthane | – | 1,20 |
| dichloracétylène | 20 ppm | ≤ 1 ppm |

Au mélange réactionnel obtenu après 6 heures de réaction, on ajoute 2,5 g de phosphate de tributyle, puis on distille. Après élimination des fractions de tête et de queue, on récupère 1600 g de dichloro-1,1 fluoro-1 éthane de titre supérieure à 99,5 %.

## EXEMPLE 6

Dans un réacteur, on place 100 g de dichloro-1,1 fluoro-1 éthane contenant 0,22 % en poids de dichloro-1,1 éthylène, puis on ajoute 210 mg de $FeCl_3$. On porte le mélange à -3°C et le sature avec du HCl gazeux.

Au bout de 2 heures, l'analyse chromatographique d'un prélèvement indique une concentration de 0,13 % en dichloro-1,1 éthylène et l'apparition d'un pic de trichloro-1,1,1 éthane.

L'essai est poursuivi pendant 10 heures et on constate que la concentration en dichloro-1,1 éthylène est alors de 0,05 %.

## EXEMPLE 7

Dans un réacteur, on place 100 g de dichloro-1,1 fluoro-1 éthane contenant 0,14 % en poids de dichloro-1,1 éthylène. On porte à -10°C, puis on ajoute 400 mg de $SbCl_5$ et on sature par HCl gazeux.

Au bout d'une heure, un prélèvement montre que la concentration en dichloro-1,1 éthylène est inférieure à 0,02 %. Du trichloro-1,1,1 éthane et du chloro-1 difluoro-1,1 éthane apparaissent dans le chromatogramme.

## EXEMPLE 8

Un débit de 100 litres/heure du soutirage réactionnel d'une fabrication de dichloro-1,1 fluoro-1 éthane et de chloro-1 difluoro-1,1 éthane est porté à la pression de 10 bars effectifs et à la température de 9°C pendant environ 3 heures, tout en injectant une très faible quantité de chlore (environ 200 g/h) pour maintenir le catalyseur $SbCl_5$ sous sa forme active.

Les compositions initiale et finale sont indiquées en poids dans le tableau suivant :

5

| COMPOSITION : | Initiale | finale |
|---|---|---|
| Produits organiques saturés (*) | 97 % | 97,3 % |
| Dichloro-1,1 éthylène | 2500 ppm | ≤ 70 ppm |
| Dichloroacétylène | 10-20 ppm | ≤ 1 ppm |
| $SbCl_5$ | 7200 ppm | 7200 ppm |
| HCl + HF | 2 % | 1,9 % |
| (*) Principalement dichloro-1,1 fluoro-1 éthane, chloro-1 difluoro-1,1 éthane et trichloro-1,1,1 éthane | | |

Dans cet exemple, la forte teneur en hydracides (HCl + HF), la pression et la basse température ont permis la rétrogradation des produits insaturés en produits saturés, en présence du catalyseur $SbCl_5$ dont l'activité a été maintenue par l'injection de chlore.

## Revendications

1. Procédé de purification d'un dichloro-1,1 fluoro-1 éthane brut contenant du dichloro-1,1 éthylène et/ou du dichloracétylène, caractérisé en ce qu'il consiste à soumettre le dichloro 1,1 fluoro-1 éthane brut à l'action du chlore et/ou de l'acide chlorhydrique, ce traitement étant réalisé en présence d'un acide de Lewis et éventuellement d'acide fluorhydrique, puis à distiller le produit ainsi traité.

2. Procédé selon la revendication 1, dans lequel le rapport molaire

$$\frac{chlore\ et/ou\ HCl}{CH_2 = CCl_2}$$

est compris entre 1,2 et 10, de préférence entre 3 et 7.

3. Procédé selon la revendication 1 ou 2, dans lequel le traitement est effectué à une température allant de -20 à +70°C, de préférence entre -5 et +30°C.

4. Procédé selon l'une des revendications 1 à 3, dans lequel l'acide de Lewis est employé en une proportion allant de 0,001 à 1 % en poids par rapport au produit brut à traiter.

5. Procédé selon l'une des revendications 1 à 4, dans lequel le traitement est effectué en continu.

## Patentansprüche

1. Verfahren zur Reinigung von technischem 1,1-Dichlor-1-fluorethan, enthaltend 1,1-Dichlorethylen und/oder Dichlorazetylen, dadurch gekennzeichnet, daß es darin besteht, das technische 1,1-Dichlor-1-fluorethan mit Chlor und/oder Salzsäure zu behandeln - diese Verarbeitung erfolgt in Gegenwart einer Lewis-Säure und gegebenenfalls von Fluorwasserstoff -und dann das so behandelte Produkt zu destillieren.

2. Verfahren nach Anspruch 1, in dem das molare Verhälnis

$$\frac{Chlor\ und/oder\ HCl}{CH_2 = CCl_2}$$

zwischen 1,2 und 10, vorzugsweise zwischen 3 und 7 liegt.

3. Verfahren nach Anspruch 1 oder 2, in dem die Verarbeitung bei einer Temperatur zwischen -20 und +70 °C, vorzugsweise zwischen -5 und +30 °C durchgeführt wird.

4. Verfahren nach einem der Ansprüche 1 bis 3, in dem die Lewis-Säure im Verhältnis von 0,001 bis 1 Gew.-% in bezug auf das zu verarbeitende technische Produkt eingesetzt wird.

5. Verfahren nach einem der Ansprüche 1 bis 4, in dem die Verarbeitung kontinuierlich durchgeführt wird.

**Claims**

1. Process for the purification of a crude 1,1-dichloro-1-fluoroethane containing 1,1-dichloroethylene and/or dichloroacetylene, characterized in that it consists in subjecting the crude 1,1-dichloro-1-fluoroethane to the action of chlorine and/or of hydrochloric acid, this treatment being carried out in the presence of a Lewis acid and optionally of hydrofluoric acid, and in then distilling the product treated in this way.

2. Process according to Claim 1, in which the molar ratio
$$\frac{\text{chlorine and/or HCL}}{\text{CH}_2 = \text{CCl}_2}$$
is between 1.2 and 10, preferably between 3 and 7.

3. Process according to Claim 1 or 2, in which the treatment is carried out at a temperature ranging from - 20 to +70°C, preferably between -5 and +30°C.

4. Process according to one of Claims 1 to 3, in which the Lewis acid is employed in a proportion ranging from 0.001 to 1 % by weight relative to the crude product to be treated.

5. Process according to one of Claims 1 to 4, in which the treatment is carried out continuously.